# EUROPÄISCHE PATENTSCHRIFT

(11) **EP 3 927 412 B1**
(45) Veröffentlichungstag und Bekanntmachung des Hinweises auf die Patenterteilung: **13.09.2023**
(21) Anmeldenummer: 20706234.0
(22) Anmeldetag: 19.02.2020
(51) Int. Cl.: A61M 37/00

(54) **APPLIKATOR FÜR MIKRONADELPFLASTER**
APPLICATOR FOR MICRONEEDLE PATCH
APPLICATEUR POUR TIMBRE À MICRO-AIGUILLES

(30) Priorität: 21.02.2019 DE 102019001251
(43) Veröffentlichungstag der Anmeldung: 29.12.2021
(73) Patentinhaber: LTS Lohmann Therapie-Systeme AG, 56626 Andernach (DE)
(72) Erfinder: BEE, Markus, 56220 Kettig Rheinland-Pfalz (DE); HACKBARTH, Ronald, 56075 Koblenz Rheinland-Pfalz (DE)
(74) Vertreter: dompatent von Kreisler Selting Werner - Partnerschaft von Patent- und Rechtsanwälten mbB
(86) Internationale Anmeldenummer: PCT/EP2020/054362
(87) Internationale Veröffentlichungsnummer: WO 2020/169667

(56) Entgegenhaltungen:
- EP-A1- 3 421 074
- WO-A1-2013/038890
- WO-A2-2007/002521
- WO-A2-2008/091602
- US-A1- 2014 276 580
- US-A1- 2018 177 991

## Beschreibung

Die Erfindung betrifft einen Applikator mit einem Griffstück und mit einem mit dem Griffstück verbundenen Druckkörper für ein Mikronadelpflaster sowie eine Einheit aus einem derartigen Applikator und einem Mikronadelpflaster mit einer Vielzahl von Mikronadeln.

Aus der US 2018/0177991 A1 ist ein Applikator für ein Mikronadelpflaster bekannt. Die Nadeln werden beim Abrollen des Stempels mittels des variierenden Normalkraftanteils in die Haut des Patienten eingedrückt.

Der vorliegenden Erfindung liegt die Aufgabe zugrunde, ein weitgehend gleichförmiges Einpressen der Mikronadeln zu ermöglichen.

Diese Problemstellung wird mit den Merkmalen des Hauptanspruchs gelöst. Dazu ist der Druckkörper relativ zum Griffstück in einem Schubgelenk oder in einem Schwenkgelenk zumindest zwischen einer Ruhestellung und einer Gebrauchsstellung mittels äußerer Betriebskräfte und einer internen Rückstellvorrichtung verstellbar gelagert. Der Druckkörper weist eine vom Schwenkgelenk oder Schubgelenk beabstandete Andruckfläche auf. In der Ruhestellung ist der Druckkörper von äußeren Betriebskräften unbelastet und die Rückstellvorrichtung weist die geringste innere Energie auf. Außerdem ist der Druckkörper mittels an der Andruckfläche angreifenden äußeren Betriebskräften relativ zum Griffstück in Richtung der Gebrauchsstellung unter Erhöhung der inneren Energie der Rückstellvorrichtung verstellbar.

In der Einheit aus dem Applikator und dem Mikronadelpflaster beträgt der kleinste Radius der Andruckfläche mindestens dem anderthalbfachen Produkt aus der Kreiszahl π und der Länge des Nadelfußes in der Pflasterlängsrichtung. Diese Länge ist z.B. bei einer kegelförmigen Mikronadel der Durchmesser des Nadelfußes. Bei einer pyramidenförmigen Mikronadel entspricht diese Länge je nach Anordnung der Mikronadel zur Pflasterlängsrichtung z.B. der Kantenlänge oder der Diagonalen des Nadelfußes. Auch andere Gestalten der Mikronadeln sind denkbar.

Der Druckkörper ist relativ zum Griffstück schwenkbar oder längsverschieblich gelagert. In der Ruhestellung wirken die geringsten Kräfte auf den Druckkörper und auf das Schub- oder Schwenkgelenk. Die Rückstellvorrichtung ist entlastet.

Sobald der Bediener den Applikator auf das Mikronadelpflaster drückt und/oder den Applikator entlang des Mikronadelpflasters z.B. zieht, greift eine äußere Betriebskraft an der Andruckfläche an. Der Druckkörper wird relativ zum Griffstück in Richtung der Gebrauchsstellung geschwenkt oder geschoben. Hierbei wird die Rückstellvorrichtung belastet. Die beim Belasten der Rückstellvorrichtung erzeugte Rückstellkraft korreliert mit der vom Bediener aufgebrachten Kraft und mit der Anpresskraft des Applikators auf das Mikronadelpflaster. Damit werden beim Ziehen oder Schieben des Applikators entlang des Mikronadelpflasters die Mikronadeln gleichmäßig in die Haut eingepresst und mit Hilfe eines Überpflasters befestigt.

Als Druckkörper kann ein Gleitkörper oder ein Wälzkörper eingesetzt werden. Der Druckkörper hat eine gewölbte Andruckfläche. Die Wölbung ist stetig ausgebildet. Sie kann einen konstanten Radius aufweisen oder aus mehreren Bereichen unterschiedlicher Radien zusammengesetzt sein. Sämtliche Radiusmittellinien der Andruckfläche liegen parallel zueinander. Beispielsweise liegt jede Radiusmittellinie in einer Normalenebene zu einer Längsrichtung des Griffstücks. Zumindest in der Gebrauchsstellung ist zumindest die Wölbungsradius-Mittellinie mit dem kleinsten Wölbungsradius windschief zur Längsachse des Griffstücks. Die Wölbungsradius-Mittellinie hat damit keinen Schnittpunkt mit der Längsachse.

Der kleinste Radius der Andruckfläche wird in Abhängigkeit der Geometrie der Mikronadeln des Mikronadelpflasters gewählt. Hiermit kann ein Verkanten der Mikronadeln beim Einbringen in die Haut verhindert werden.

Weitere Einzelheiten der Erfindung ergeben sich aus den Unteransprüchen und den nachfolgenden Beschreibungen schematisch dargestellter Ausführungsbeispiele.
Figur 1: Applikator in der Ruhestellung;
Figur 2: Applikator in der Gebrauchsstellung;
Figur 3: Rückansicht des Applikators;
Figur 4: Applikator beim Fixieren des Mikronadelpflasters auf der Haut;
Figur 5: Einheit aus Applikator und Mikronadelpflaster;
Figur 6: Schnitt des Druckkörpers der Einheit aus Figur 5;
Figur 7: Einheit aus Figur 5 beim Aufbringen des Mikronadelpflasters;
Figur 8: Detail des Mikronadelpflasters mit der Abdeckfolie;
Figur 9: Applikator mit Schubgelenk;
Figur 10: Applikator mit Spendiervorrichtung in der Bereitschaftsstellung;
Figur 11: Applikator aus Figur 10 beim Applizieren;
Figur 12: Applikator aus Figur 10 nach dem Aufbringen des Mikronadelpflasters.

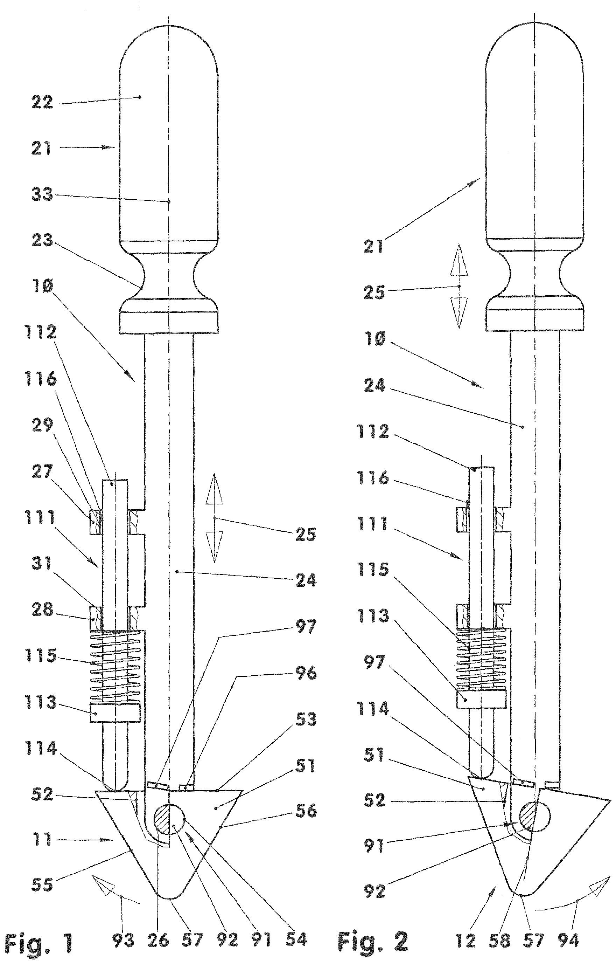

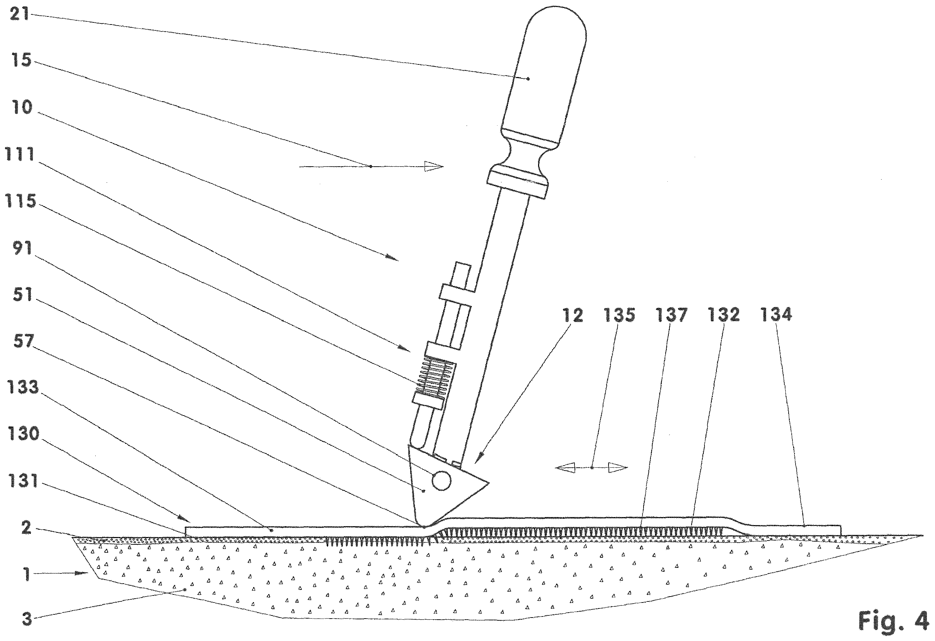

Die Figur 1 zeigt stark vereinfacht einen Applikator (10) in einer Ruhestellung (11). Die Figur 2 zeigt diesen Applikator (10) in einer Gebrauchsstellung (12). Derartige Applikatoren (10) werden eingesetzt, um Mikronadelpflaster (130) auf der Haut (1) eines Patienten zu fixieren, vgl. Figur 4. Das einzelne Mikronadelpflaster (130) hat auf der dem Applikator (10) abgewandten Unterseite (131) eine Vielzahl z.B. geometrisch identisch ausgebildeter Mikronadeln (132). Diese Mikronadeln (132) werden mit einem hier nicht dargestellten Backing Layer zusammengehalten, der von einem Überpflaster (133) überdeckt ist. Bei der Applikation werden die Mikronadeln (132) mit Hilfe des Applikators (10) in die Haut (1) eingedrückt oder eingepresst. Nach dem Befestigen des Mikronadelpflasters (130) auf der Haut (1) können z.B. im Mikronadelpflaster (130) enthaltene Wirkstoffe in unter der Hornhaut (2) liegende Hautschichten (3) abgegeben werden.

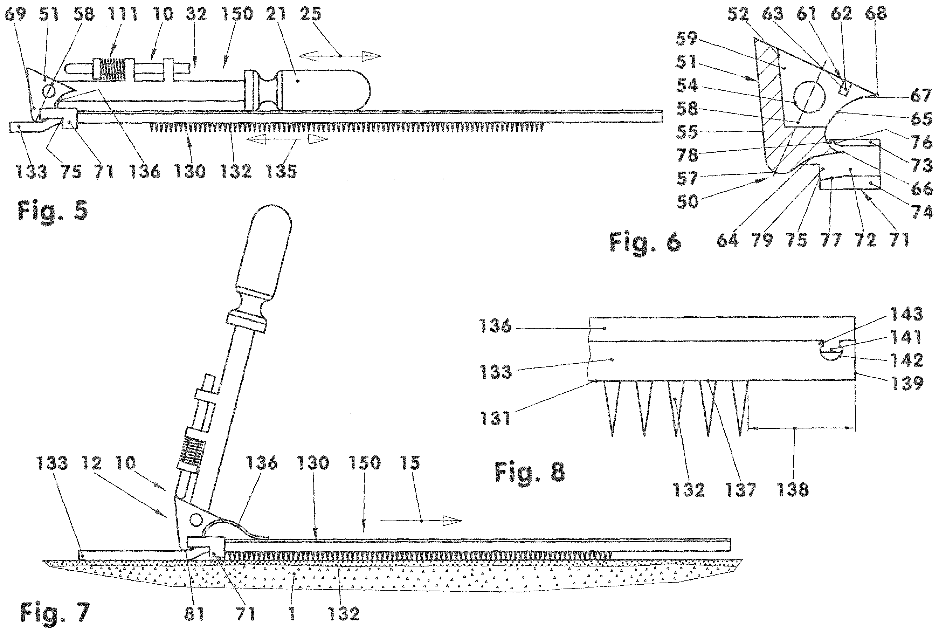

Das Überpflaster (133), vgl. Figur 4, steht in der Pflasterlängsrichtung (135) und in der normal hierzu orientierten Pflasterquerrichtung über den Bereich der Mikronadeln (132) über. Im Lieferzustand, vgl. z.B. Figur 8, ist das Überpflaster mittels einer hier nicht dargestellten Sterilbarriere und einer Abdeckfolie (136), z.B. eines Release liners, abgedeckt. Sowohl die Sterilbarriere als auch die Abdeckfolie (136) sind vor dem Applizieren des Mikronadelpflasters (130) zu entfernen.

Der in den Figuren 1 - 4 dargestellte Applikator (10) umfasst ein Griffstück (21) und einen mit diesem verbundenen Druckkörper (51). In allen Darstellungen ist das Griffstück (21) im Vergleich zum Druckkörper (51) und zum Mikronadelpflaster (130) verkleinert dargestellt. Die Verbindung des Druckkörpers (51) mit dem Griffstück (21) ist im Ausführungsbeispiel als Schwenkgelenk (91) mit einer Rückstellvorrichtung (111) ausgebildet.

Das Griffstück (21) hat einen Handballenkörper (22) und eine Fingerausnehmung (23). Dieser Bereich des Griffstücks (21) ist beispielsweise rotationssymmetrisch in Bezug auf eine Längsachse (33) des Griffstücks (21) ausgebildet. Die Fingerausnehmung (23) ist in diesem Ausführungsbeispiel eine umlaufende Rinne. Weiterhin hat das Griffstück (21) eine Tragstange (24) Diese hat im Ausführungsbeispiel eine weitgehend quadratische Querschnittsfläche. Die Mittellinie der Tragstange (24) liegt z.B. in der Längsachse (33) des Griffstücks (21). An ihrem dem Handballenkörper (22) abgewandten Ende hat die Tragstange (24) eine Schwenkbolzenaufnahme (26). In dieser Schwenkbolzenaufnahme (26) ist im Ausführungsbeispiel ein Schwenkbolzen (92) gelagert, der den Druckkörper (51) durchdringt. Die die Mittellinie der Schwenkbolzenaufnahme (26) bildende Schwenkachse (95) ist in der Ruhestellung (11) z.B. normal zur Längsachse (33) des Griffstücks (21) orientiert. Die Schwenkachse (95) kann in der Ruhestellung (11) auch windschief zur Längsachse (33) ausgerichtet sein. Der Druckkörper (51) ist somit mittels eines Schwenkgelenks (91) relativ zum Griffstück (21) schwenkbar gelagert. Das Schwenkgelenk (91) hat zwei einander entgegengesetzt orientierte Freiheitsgrade (93, 94). Die beiden Schwenkfreiheitsgrade (93, 94) sind in diesem Ausführungsbeispiel mittels Anschlägen (96, 97) begrenzt. In der Darstellung der Figur 1 begrenzt ein erster Anschlag (96) den Schwenkbereich des Druckkörpers (51) relativ zum Griffstück (21) in der Ruhestellung (11). Dieser erste Anschlag (96) bildet ein Sicherungselement (96) des Applikators (10) in der Ruhestellung (11). Gemäß der Figur 2 wird der Schwenkbereich des Druckkörpers (51) relativ zum Griffstück (21) in der Gebrauchsstellung (12) mittels des zweiten Anschlags (97) begrenzt. Der Applikator (10) kann auch ohne den zweiten Anschlag (97) ausgebildet sein. Sowohl der erste Anschlag (96) als auch der zweite Anschlag (97) können auch anders angeordnet sein.

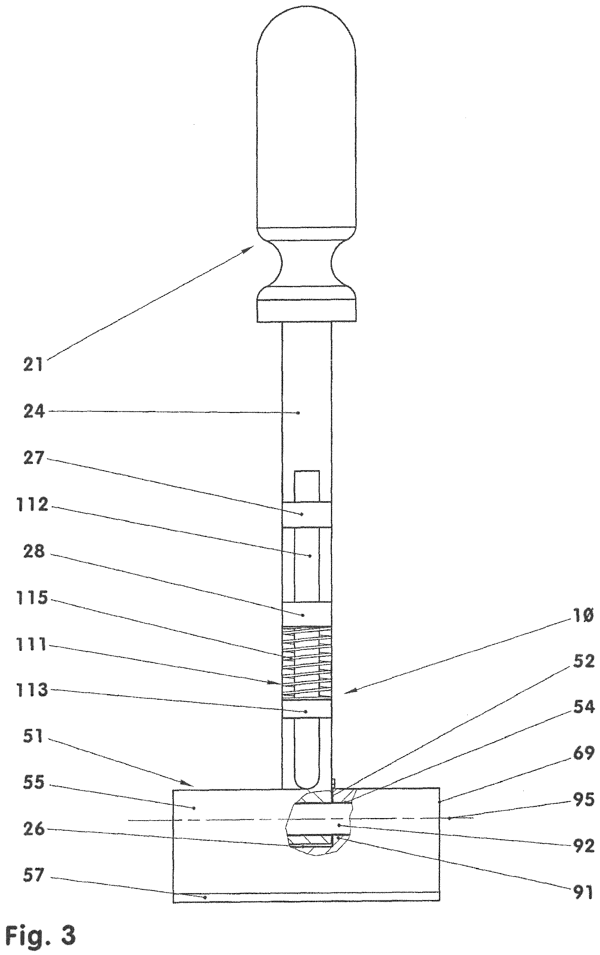

Der Druckkörper (51) ist z.B. prismenförmig aufgebaut. Im Ausführungsbeispiel entspricht seine normal zur Schwenkachse (95) orientierte Querschnittsfläche zumindest annähernd der Fläche eines gleichseitigen Dreiecks. Die Querschnittsfläche kann auch mehr als drei Ecken oder Abrundungen aufweisen. Sie kann auch unsymmetrisch ausgebildet sein. Im mittleren Bereich der Schwenkachse (95), vgl. Figur 3, hat der Druckkörper (51) eine Griffstückaufnahme (52). In dieser als Aussparung ausgebildeten Griffstückaufnahme (52) ist das Griffstück (21) gelagert. Es ist auch denkbar, dass das Griffstück (21) den Druckkörper (51) an dessen beiden z.B. zueinander parallelen Stirnseiten greift. Im Ausführungsbeispiel hat der Druckkörper (51) eine z.B. ebene Oberseite (53). An dieser Oberseite (53) liegt in der Ruhestellung (11) der erste Anschlag (96) und in der Gebrauchsstellung (12) der zweite Anschlag (97) an. Die Schwenkbolzenausnehmung (54) des Druckstücks (51) ist in den Darstellungen der Figuren 1 und 2 in Richtung der Oberseite (53) versetzt zur geometrischen Mittellinie der Querschnittsfläche des Druckkörpers (51).

Die Oberseite (53) wird im Ausführungsbeispiel von zwei Flanken (55, 56) begrenzt. Dies sind z.B. ebene Flächen, die mittels einer der Oberseite (53) abgewandten Andruckfläche (57) miteinander verbunden sind. Die Andruckfläche (57) ist in diesem Ausführungsbeispiel eine einachsig gewölbte Fläche. Die Mittellinie der Wölbung durchdringt den Druckkörper (51) parallel zur Schwenkachse (95). Der Wölbungsradius beträgt in diesem Ausführungsbeispiel 5 Millimeter. Die Andruckfläche (57) kann auch aus mehreren Wölbungsabschnitten zusammengesetzt sein. Die Mittellinien dieser Wölbungsabschnitte, die z.B. die gesamte Breite des Druckkörpers (51) umfassen, liegen dann parallel zueinander. Die Übergänge zwischen den einzelnen Wölbungsabschnitten sind stetig ausgebildet. Auch die Übergänge der Andruckfläche (57) zu den Flanken (55, 56) können stetig ausgebildet sein.

An der Tragstange (24) ist im Ausführungsbeispiel eine Halterung (27, 28) für die Rückstellvorrichtung (111) angeordnet. Diese Halterung (27, 28) umfasst z.B. zwei an der Tragstange (24) befestigte Führungslaschen (27, 28). Diese Führungslaschen (27, 28) können auch Teile der Tragstange (24) sein. Sie sind in der Längsrichtung (25) des Griffstücks (21) zueinander beabstandet. Im Ausführungsbeispiel beträgt der Abstand der Führungslaschen (27, 28) zueinander 15 % der Länge der Tragstange (24). Jede der Führungslaschen (27; 28) hat einen Durchbruch (29; 31). Beispielsweise fluchten beide Durchbrüche (29, 31) miteinander. Die Querschnittsfläche des einzelnen Durchbruchs (29; 31) kann kreisförmig, rechteckig, sechseckig, etc. ausgebildet sein.

Die Rückstellvorrichtung (111) umfasst eine in den Führungslaschen (27, 28) gelagerte Druckstange (112) und einen auf der Druckstange (112) sitzenden Federenergiespeicher (115). Die in den Durchbrüchen (29, 31) steckende Druckstange (112) liegt parallel zur Längsrichtung (25) des Griffstücks (21). Ihr oberes Ende ragt aus der oberen Führungslasche (27) heraus. Unterhalb der unteren Führungslasche (28) hat die Druckstange (112) einen Stützbund (113). Dieser hat beispielsweise die Gestalt eines umlaufenden Ringbunds. Das untere, in den Figuren 1 und 2 den Druckkörper (51) kontaktierende Druckende (114) der Druckstange (112) ist im Ausführungsbeispiel halbkugelförmig ausgebildet.

Im oberen Bereich hat die Druckstange (112) einen Indikator (116), z.B. eine Markierung (116). Dies ist beispielsweise ein Farbring, ein Punkt, eine Kerbe, etc. In der Darstellung der Figur 1 liegt diese Markierung (116) innerhalb der oberen Führungslasche (27). In der in der Figur 2 gezeigten Gebrauchsstellung (12) steht die Markierung (116) oberhalb der oberen Führungslasche (27). Anstatt einer einzelnen Markierung (116) ist es denkbar, z.B. untereinander abgeordnete verschiedenfarbige Markierungen auf der Druckstange (112) anzubringen. Beispielsweise könnte die oberste Markierung rot, eine mittlere Markierung gelb und die unterste Markierung (116) grün sein. Anstatt des beschriebenen optischen Signals mittels der Markierung (116) kann der Applikator (10) auch ein haptisches, akustisches, etc. Signal an den Bediener geben.

Mit dem Stützbund (113) trägt die Druckstange (112) den Federenergiespeicher (115). Dieser ist im Ausführungsbeispiel als Druckfeder (115) ausgebildet. Die Druckfeder (115) sitzt in der Darstellung der Figur 1 auf einen Restenergiewert vorgespannt zwischen dem Stützbund (113) und der unteren Führungslasche (28). Diese eingeprägte Kraft der Rückstellvorrichtung (111) drückt in diesem Ausführungsbeispiel in der Ruhestellung (11) die Druckstange (112) auf den am Sicherungselement (96) anliegenden Druckkörper (51).

Der Federenergiespeicher (115) kann auch am Stützbund (113) und an der unteren Führungslasche (28) z.B. formschlüssig fixiert sein. Weiterhin kann die Druckstange (112) mit ihrem Druckende (114) z.B. mittels eines Schub-Schwenkgelenks mit dem Druckkörper (51) verbunden sein. Bei derartigen Ausführungen können z.B. die Anschläge (96, 97) entfallen.

Bei einer Bewegung des Druckkörpers (51) aus der Ruhestellung (11) in Richtung der Gebrauchsstellung (12) wird der Druckkörper (51) um die Schwenkachse (95) geschwenkt. Die Oberseite (53) des Druckkörpers (51) drückt auf das Duckende (114) der Druckstange (112). Die Druckstange (112) wird nach oben verdrängt. Hierbei komprimieren der Stützbund (113) und die untere Führungslasche (28) z.B. hysteresefrei die Druckfeder (115). Die innere Energie der Rückstellvorrichtung (111) steigt an. Gleichzeitig wandert die Markierung (116) nach oben, sodass beispielsweise der grüne Bereich oberhalb der oberen Führungslasche (27) sichtbar wird. Beim Entlasten des Druckkörpers (51) schnellt dieser wieder unter Entladung des Federenergiespeichers (115) in die Ruhestellung (11) zurück.

Die Rückstellvorrichtung (111) kann als Federenergiespeicher (115) auch eine Zugfeder, eine Schrauben-Drehfeder, eine kegelige Schrauben-Druckfeder, ein Tellerfederpaket, etc. aufweisen. Alle diese Federenergiespeicher (115) sind so ausgebildet, dass in der Ruhestellung (11) die Rückstellvorrichtung (111) die geringste innere Energie hat. Der Federenergiespeicher (115) kann hierbei vollständig entlastet sein oder eine geringe Vorspannung aufweisen. Beim Einsatz eines Federenergiespeichers (115) mit Vorspannung wird der Druckkörper (51) relativ zum Griffstück (21) ständig in Richtung der Ruhestellung (11) belastet. Bei einer Bewegung des Druckkörpers (51) in Richtung der Gebrauchsstellung (12) wird der jeweilige Federenergiespeicher (115) zusätzlich belastet. Seine innere Energie wird erhöht. Damit steigt auch die Rückstellkraft an.

Beispielsweise beim Einsatz einer Schrauben-Drehfeder bzw. Schenkelfeder umgreift diese die Schwenkachse (95) bzw. den Schwenkbolzen (92). Beispielsweise ist ein Schenkel der Schenkelfeder am Druckkörper (51) und der andere Schenkel am Griffstück (21) fixiert, z.B. geklemmt. In der Ruhestellung (11) ist die Schenkelfeder z.B. unbelastet. Die Position des Druckkörpers (51) relativ zum Griffstück (21) wird auch ohne den ersten Anschlag (96) gehalten. Sobald der Druckkörper (51) aus dieser Lage ausgeschwenkt wird, werden die Schenkel der Schenkelfeder relativ zueinander um die Schwenkachse (95) verschwenkt. Die Schenkelfeder wird belastet, wobei die innere Energie der Rückstellvorrichtung (111) zunimmt. Der Drucckörper (51) kann wie oben beschrieben oder beispielsweise als Rolle ausgebildet sein. In diesem Ausführungsbeispiel kann ein Indikator (116) beispielsweise die Winkellage des Druckkörpers (51) relativ zum Griffstück (21) anzeigen.

Vor dem Einsatz des in den Figuren 1 bis 4 dargestellten Applikators (10) wird das Mikronadelpflaster (130) auf die Haut (1) des Patienten aufgelegt. Nach dem Entfernen der Abdeckfolie (136) wird der Applikator (10) z.B. in Pflasterlängsrichtung (135) über das Mikronadelpflaster (130) gezogen, vgl. Figur 4. Beim Ziehen stellt der Bediener den Applikator (10) schräg. Er drückt ihn so gegen das Mikronadelpflaster (130), dass zunächst der in der Pflasterlängsrichtung (135) hintere Rand des Überpflasters (133) an die Haut (1) des Patienten angepresst wird. Die Breite des Druckkörpers (51) in der Pflasterquerrichtung ist größer oder gleich der Breite des Mikronadelpflasters (130). Das Mikronadelpflaster (130) wird auf der ganzen Breite in der Pflasterquerrichtung (135) auf der Haut (1) fixiert. Hiermit werden z.B. Lufteinschlüsse minimiert-

Beim Ziehen des Applikators (10) und Anpressen des Druckkörpers (51) an das Mikronadelpflaster (130) gleitet der Druckkörper (51) mit seiner Andruckfläche (57) entlang der Oberfläche (134) des Überpflasters (133). Auf die Andruckfläche (57) wirkt eine äußere Betriebskraft. Diese äußere Betriebskraft ist entgegen der Zugrichtung (15) des Applikators (10) orientiert. Zumindest im Bereich des Überpflasters (133) ist die äußere Betriebskraft auch parallel zur Oberfläche (134) des Mikronadelpflasters (130) orientiert. Die äußere Betriebskraft verzögert damit die Bewegung des Applikators (10). Der Druckkörper (51) wird um die Schwenkachse (95) des Schwenkgelenks (91) in Richtung der Gebrauchsstellung (12) geschwenkt. Der Federenergiespeicher (115) wird in Abhängigkeit der Höhe der Anpresskraft des Druckkörpers (51) auf das Mikronadelpflaster (130) belastet. Beispielsweise wird die grüne Markierung (116) sichtbar. Dieses Signal bedeutet für den Bediener, dass die Anpresskraft z.B. genügend groß ist. Der Bediener kann das Befestigen des Mikronadelpflasters (130) fortsetzen. Der Druckkörper (51) befindet sich nun relativ zum Griffstück (21) in der Gebrauchsstellung (12) oder in einem an die Gebrauchsstellung (12) angrenzenden Bereich.

Beim weiteren schleppenden Ziehen des Applikators (10) entlang des Mikronadelpflasters (130) werden die einzelnen Mikronadeln (132) des Mikronadelpflasters (130) in die Haut (1) eingedrückt. Beispielsweise beträgt der Radius der Andruckfläche (57) mindestens das 1,5 -fache des Produkts aus der Kreiszahl π und dem Durchmesser eines Nadelfußes (137). Im Ausführungsbeispiel erfolgt das Eindrücken der Mikronadeln (132) zeilenweise. Es ist aber auch denkbar, den Applikator (10) z.B. diagonal über das Mikronadelpflaster (130) zu ziehen. Der Bediener kann die Anpresskraft mittels der optischen, haptischen oder akustischen Anzeige weiterhin überwachen. Wird die Anpresskraft zu niedrig, wenn also z.B. die grüne Markierung (116) unsichtbar wird, kann er die Anpresskraft durch stärkeres Drücken auf das Griffstück (21) wieder erhöhen.

Beim Fixieren des Mikronadelpflasters (130) mittels des Applikators (10) wird auch das Überpflaster (133) an der Haut (1) des Patienten befestigt. Nach der Applikation kann der Applikator (10) entweder erneut eingesetzt werden oder entsorgt werden. Beim Abnehmen des Applikators (10) vom Mikronadelpflaster (130) und von der Haut (1) des Patienten entlastet sich der komprimierte Federenergiespeicher (115).

Die Druckfeder (115) längt sich. Mittels der freigesetzten Energie der Rückstellvorrichtung (111) wird der Druckkörper (51) relativ zum Griffstück (21) in die Ruhestellung (11) zurückgestellt.

Der Einsatz des Applikators (10) mit einer anders aufgebauten Rückstellvorrichtung (111) erfolgt analog. Bei Belastung des Applikators (10) teilt sich die vom Bediener aufgebrachte Kraft relativ zum Mikronadelpflaster (130) in eine Tangentialkomponente entlang des Mikronadelpflasters (130) und eine Normalkomponente in Richtung des Mikronadelpflasters (130) auf. Jede einzelne dieser Kraftkomponenten und damit auch die Resultierende dieser Komponenten bewirkt ein Schwenken des Drückkörpers (51) relativ zum Griffstück (21) in Richtung der Gebrauchsstellung (12). Beispielsweise kann der erreichte Schwenkwinkel auch in diesem Ausführungsbeispiel mittels einer Anzeige überwacht werden. Hiermit wird eine Mindestanpresskraft zum zuverlässigen Einbringen der Mikronadeln (132) sichergestellt. Beim Einsatz eines als Rolle ausgebildeten Druckkörpers (51) kann dieser auf dem Mikronadelpflaster (130) abwälzen und/oder auf diesem entlang gleiten.

Die Figuren 5 - 7 zeigen eine Einheit (150) aus einem Applikator (10) und einem Mikronadelpflaster (130) sowie den Druckkörper (51) als Einzelteil. Die Einheit (150) wird beispielsweise zum einmaligen Gebrauch geliefert. In dieser Einheit (150) trägt das Mikronadelpflaster (130) auf dem Überpflaster (133) die Abdeckfolie (136). In dem in der Figur 5 dargestellten Lieferzustand ist am Rand des Überpflasters (133) und der Abdeckfolie (136) der Applikator (10) angeordnet, dessen Griffstück (21) auf der Abdeckfolie (136) aufliegt.

Der Applikator (10) ist in diesem Ausführungsbeispiel weitgehend so aufgebaut, wie im Zusammenhang mit dem in den Figuren 1 - 4 dargestellten Ausführungsbeispiel beschrieben. Der Federenergiespeicher (115) ist z.B. formschlüssig sowohl mit dem Stützbund (113) als auch mit der unteren Führungslasche (28) verbunden. Das Griffstück (21) liegt in einer Vertriebslage (32), bei der seine Längsrichtung (25) einen Winkel von z.B. 65 Grad zu einer durch die Schwenkachse (95) und die Radiusmittellinie der Andruckfläche (57) aufgespannten Verbindungsebene (58) einschließt. Den gleichen Winkel schließt beispielsweise die Verbindungsebene (58) in dieser Darstellung mit der Pflasterlängsrichtung (135) ein. Das Verhältnis des Radius der Andruckfläche (57) zum Durchmesser des Nadelfußes (137) bzw. zur Länge des Nadelfußes (137) in der Pflasterlängsrichtung (135) entspricht in diesem Ausführungsbeispiel dem oben genannten Verhältnis.

In der Figur 6 ist ein Längsschnitt des Druckkörpers (51) dieses Applikators (10) dargestellt. Die Schnittebene dieser Darstellung liegt in der vertikalen Mittenlängsebene des Applikators (10). Die Schwenkbolzenausnehmung (54), die Andruckfläche (57), die hintere Flanke (55) und die Oberseite (53) sind so ausgebildet, wie im Zusammenhang mit dem ersten Ausführungsbeispiel beschrieben. Die Griffstückaufnahme (52) ist zur vorderen Flanke (56) hin offen. Damit ist das Griffstück (21) in der Vertriebslage (32) z.B. ohne Anschlag gelagert.

In der Griffstückaufnahme (52) ist in diesem Ausführungsbeispiel an mindestens einer parallel vertikalen Mittenlängsebene orientieren Ausnehmungsfläche (59) ein Rast- oder Keilgesperre (61) angeordnet. Dieses umfasst z.B. einen elastisch verformbaren Keil (62), dessen Dicke von der vorderen Flanke (56) in Richtung der hinteren Flanke (55) ansteigt. Die in Richtung der Verbindungsebene (58) zeigende Keilfläche (63) ist eine Rastfläche. Beim Schwenken des Griffstücks (21) aus der Vertriebslage (32) in Richtung der Ruhelage (11) verrastet die Tragstange (24) in der Ruhelage (11) hinter der Keilfläche (63). Das Rast- oder Keilgesperre (61) blockiert damit z.B. unlösbar ein Zurückschwenken des Griffstücks (21) in die Vertriebslage (32). In der Ruhelage (11) bildet das Rast- oder Keilgesperre (61) damit ein Sicherungselement (61). Der in den Figuren 5 - 7 dargestellte Applikator (10) kann auch ohne das Rast- oder Keilgesperre (61) ausgebildet sein.

Die vordere Flanke (56) umfasst in diesem Ausführungsbeispiel zwei Leitflächen (64, 65), die voneinander durch eine Trennkante (66) getrennt sind. Die Trennkante (66) verläuft parallel zur Schwenkachse (95). Sie ragt spitzwinklig in der Zugrichtung (15) aus dem Druckkörper (51) hervor. In der Trennkante (66) schließen die beiden konkav gewölbt ausgebildeten Leitflächen (64, 65) beispielsweise einen Winkel von 10 Grad bis 20 Grad ein. Im Ausführungsbeispiel beträgt der eingeschlossene Winkel 17 Grad. Die Ebene der Winkelhalbierenden der Trennkante (66) schließt mit der Verbindungsebene (58) den gleichen Winkel ein wie Pflasterlängsrichtung (135) mit der Verbindungsebene (58).

Im Ausführungsbeispiel sind beide Leitflächen (64, 65) aus mehreren, parallel zueinander liegenden einachsigen Wölbungsabschnitten zusammengesetzt. Der Drucckörper () hat über seine gesamte Breite - mit Ausnahme der Griffstückaufnahme (52) - die gleiche Querschnittsfläche. Die gedachten Mittellinien der einzelnen Wölbungsabschnitte beider Leitflächen (64, 65) liegen parallel zur Schwenkachse (95). Diese Mittellinien sind in der Zugrichtung (15) versetzt zum Druckkörper (51). Die untere Leitfläche (64) geht stetig in die Andruckfläche (57) über. Beispielsweise nimmt ihr Wölbungsradius von der Trennkante (66) aus ab. Die untere Leitfläche (64) geht dann tangential in die entgegengesetzt gewölbte Andruckfläche (57) über.

Die obere Leitfläche (65) hat in der Darstellung der Figur 6 einen sich von der Trennkante (66) aus spiralförmig aufweitenden Krümmungsradius. Im Ausführungsbeispiel ist die Tangentialebene am oberen Auslauf (67) der oberen Leitfläche (65) parallel zur Ebene der Winkelhalbierenden der Trennkante (66). Die obere Leitfläche (65) stößt im Ausführungsbeispiel mit der Oberseite (53) des Druckkörpers (51) in einer Längskante (68) zusammen.

An jeder der Stirnseiten (69) trägt der Druckkörper (51) jeweils ein Führungselement (71). Der Druckkörper (51) und die beiden Führungselemente (71) bilden in diesem Ausführungsbeispiel eine Druckkörpergruppe (50). In dieser Druckkörpergruppe (50) sind beide spiegelbildlich zueinander ausgebildete Führungselemente (71) starr mit dem Druckkörper (51) verbunden. Beispielsweise sind sie angeformt. Es ist aber auch denkbar, die Führungselemente (71) in jeweils einem Schwenkgelenk mit dem Druckkörper (51) zu verbinden. Die bei schwenkbaren Führungselementen (71) miteinander fluchtenden Schwenkachsen beider Führungselemente (71) liegen parallel zur Radiusmittellinie der Andruckfläche (57).

Das einzelne Führungselement (71) hat eine Führungsplatte (72) und jeweils zwei in Richtung der vertikalen Mittenlängsebene des Druckkörpers (51) zeigende Führungsstege (73, 74). Die Führungsplatten (72) liegen parallel zur vertikalen Mittenlängsebene an den Außenseiten des Druckkörpers (51). Sie haben beispielsweise eine quaderförmige Hüllkontur. An ihrem unteren, in Richtung der Andruckfläche (57) zeigenden Ende haben sie eine Ausklinkung (75).

Die Führungsstege (73, 74) sind flach ausgebildet. An ihrem in Richtung der Andruckfläche (57) zeigenden Ende sind sie von innen nach außen hin bogenförmig verjüngt. Die jeweilige Mittelachse des Radius entspricht beispielsweise der Mittelachse des Wölbungsabschnittes der jeweils benachbarten Leitfläche (64; 65). Der Abstand der oberen Verjüngung (76) zur oberen Leitfläche (65) entspricht der Dicke der Abdeckfolie (136). Der Abstand der unteren Verjüngung (77) zur unteren Leitfläche (64) entspricht der Dicke des Überpflasters (133). Beide Führungsstege (73, 74) ragen beispielsweise um den Betrag des seitlichen Überstands (138) des Überpflasters (133) über die Mikronadeln (132), vgl. Figur 8, in Richtung der vertikalen Mittenlängsebene. Es ist aber auch denkbar, den oberen Führungssteg (73) durchgehend zwischen den beiden Führungsplatten (72) auszubilden.

Die Figur 8 zeigt ein Detail des Mikronadelpflasters (130) mit der Abdeckfolie (136). Beispielsweise ist der in der Pflasterlängsrichtung (135) gesehene rechte Rand des Mikronadelpflasters (130) dargestellt. An beiden Längsseiten (139) ist die Abdeckfolie (136) formschlüssig mit dem Mikronadelpflaster (130) verbunden. Hierbei hat beispielsweise die Abdeckfolie (136) eine elastisch verformbare Längsnase (141), die in eine elastisch verformbare Längsnut (142) mit Umgriff (143) des Mikronadelpflasters (130) eingreift.

In der in den Figuren 5 und 7 dargestellten Einheit (150) greift der Applikator (10) mit der Trennkante (66) in den Zwischenraum zwischen der Abdeckfolie (136) und dem Überpflaster (133). Der obere Führungssteg (73) liegt auf der Abdeckfolie (136) auf. Der untere Führungssteg (74) kontaktiert die Unterseite (131) des Überpflasters (133).

Die Abdeckfolie (136) ist mit einem freien Ende in einen oberen Leitpfad (78) zwischen der Trennkante (66) und dem oberen Führungssteg (73) und zwischen der oberen Verjüngung (76) und der oberen Leitfläche (65) eingeführt. Beispielsweise liegt die Abdeckfolie (136) bereits an der oberen Leitfläche (65) an.

Das Überpflaster (133) ist entlang des unteren Leitpfades (79) geführt. Dieser verläuft zwischen der Trennkante (66) und dem unteren Führungssteg (74) sowie zwischen der unteren Verjüngung (77) und der unteren Leitfläche (64). Im Ausführungsbeispiel ist das Überpflaster (133) in der in der Figur 5 dargestellten Vertriebslage (32) bis unter die Andruckfläche (57) geführt.

Um das Mikronadelpflaster (130) einzusetzen, wird dieses beispielsweise mit demjenigen Ende des Überpflasters (133) auf die Haut (1) des Patienten aufgeklebt, an dem sich der Applikator (10) befindet. Das Griffstück (21) wird aus der in der Figur 5 dargestellten Vertriebslage (32) ausgeklappt, bis es beispielsweise das Rast- oder Keilgesperre (61) betätigt. Der Druckkörper (51) steht nun relativ zum Griffstück (21) in der Ruhestellung (11). Der Federenergiespeicher (115) ist nicht oder nur geringfügig belastet.

Der Bediener zieht nun unter gleichzeitigem Andrücken den Applikator (10) in der Zugrichtung (15) entlang des Mikronadelpflasters (130). Das Andrücken des Applikators (10) erfolgt in der Längsrichtung (25) des Griffstücks (21). Da beim Ziehen die Längsrichtung (25) mit der Verbindungsebene (58) einen Winkel ungleich 0 Grad und 180 Grad einschließt, wirkt auf den Druckkörper (51) ein Drehmoment um die Momentanpollinie (81). Diese Momentanpollinie (81) ist die Kontaktlinie der Andruckfläche (57) mit dem Überpflaster (133). Die Andruckfläche (57) wälzt am Überpflaster (133) beim Verstellen des Druckkörpers (51) ab, wobei sich der Winkel zwischen der Längsachse (33) und der Verbindungsebene (58) weiter vergrößert. Der Federenergiespeicher (115) wird belastet, bis sich ein Gleichgewicht der Federkraft und der vom Bediener aufgebrachten Kraft einstellt. Gleichzeitig wandert beispielsweise die Markierung (116) in den sichtbaren Bereich.

Beim Ziehen des Applikators (10) entlang des Mikronadelpflasters (130) trennt die Trennkante (66) die Verbindung zwischen der Abdeckfolie (136) und dem Überpflaster (133) auf. Die Abdeckfolie (136) wird entlang der oberen Leitfläche (65) abgeleitet und legt sich beispielsweise lose auf den noch nicht bearbeiteten Bereich der Abdeckfolie (136).

Das Mikronadelpflaster (130) mit dem Überpflaster (133) wird mittels der Trennkante (66) nach unten zur Andruckfläche (57) geleitet. Die Mikronadeln (132) werden z.B. zeilenweise in die Haut (1) eingedrückt. Das Überpflaster (133) wird auf die Haut (1) gedrückt und haftet dort adhäsiv.

Sobald das Mikronadelpflaster (130) auf der Haut (1) des Patienten fixiert ist, ist die Abdeckfolie (136) vollständig entfernt. Der Applikator (10) hat sich vom Mikronadelpflaster (130) gelöst.

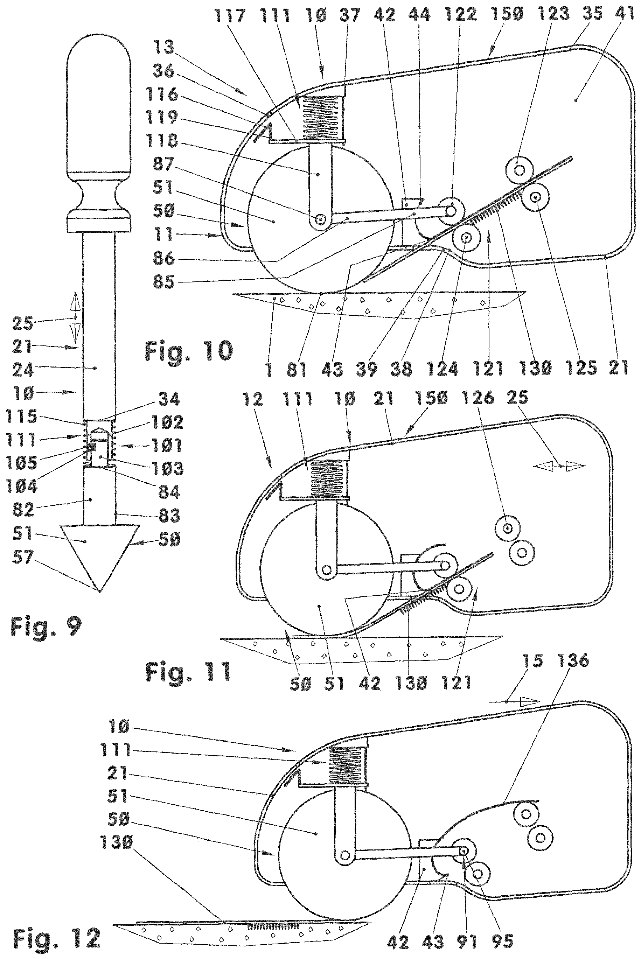

Die Figur 9 zeigt einen Applikator (10) mit einem Schubgelenk (101). Dieses Schubgelenk (101) verbindet eine Druckkörpergruppe (50) mit dem Griffstück (21). Das Schubgelenk (101) besteht aus einem Hohlprisma (102), in dem teleskopartig ein Vollprisma (103) geführt ist. Auf dem Schubgelenk (101) sitzt eine Rückstellvorrichtung (111), die sich am Griffstück (21) und an der Druckkörpergruppe (50) abstützt.

Das Griffstück (21) ist weitgehend so aufgebaut, wie im Zusammenhang mit dem ersten Ausführungsbeispiel beschrieben. Die Tragstange (24) hat einen Bereich kreisförmigen Querschnitts, der an einem z.B. umlaufenden Haltebund (34) endet. An diesem Haltebund (34) ist zentral das Hohlprisma (102) angeformt. Dies ist beispielsweise eine Hohlstange mit kreisförmiger Querschnittsfläche. Dieses Hohlprisma (102) hat im Ausführungsbeispiel einen langlochartigen, in der Längsrichtung (25) orientierten Längsschlitz (104).

Die Tragstange (24) kann auch eine andere, z.B. eine quadratische Querschnittsfläche aufweisen. Der Haltebund (34) kann aus einzelnen Segmenten oder Abschnitten bestehen. Auch das Hohlprisma (102) kann z.B. eine quadratische oder eine andere von einer kreisförmigen Querschnittsfläche abweichende Querschnittsfläche haben.

Die Druckkörpergruppe (50) umfasst einen Druckkörper (51) und eine am Druckkörper (51) angeordnete Führungsstange (82). Der Druckkörper (51) ist beispielsweise weitgehend so aufgebaut, wie im Zusammenhang mit dem ersten Ausführungsbeispiel beschrieben. Die Führungsstange (82) ist starr mit dem Druckkörper (51) verbunden. Sie ragt in der Längsrichtung (25) aus diesem heraus.

Die Führungsstange (82) hat im Ausführungsbeispiel einen zylindrischen Bereich (83), aus dem zentral das z.B. ebenfalls zylindrisch ausgebildete Vollprisma (103) herausragt. Den Übergang zwischen dem zylindrischen Bereich (83) und dem als zylindrische Stange ausgebildeten Vollprisma (103) bildet ein z.B. umlaufender Stützbund (84). Der Stützbund (84) kann wie der Haltebund (34) ausgebildet sein. Die Querschnittsfläche des Vollprismas (103) ist geometrisch ähnlich der Innenquerschnittsfläche des Hohlprismas (102), sodass das Hohlprisma (102) das Vollprisma (103) aufnehmen und führen kann. Im Ausführungsbeispiel hat das Vollprisma (103) einen z.B. federbelasteten Rastzapfen (105), der bei montiertem Schubgelenk (101) in den Längsschlitz (104) des Hohlprismas (102) eingreift. Damit wird beispielsweise sowohl eine Verdrehsicherung als auch eine Auszugssicherung des Schubgelenks (101) gebildet. Auch andere Ausbildungen einer Verdrehsicherung und einer Auszugssicherung sind denkbar.

Der Druckkörper (51) kann auch in diesem Ausführungsbeispiel als Rolle ausgebildet sein. Der Rollenradius entspricht beispielsweise dem oben genannten Radius der Andruckfläche (57). Der Druckkörper (51) ist dann z.B. an beiden Stirnseiten drehbar an einer gabelförmig ausgebildeten Führungsstange (82) gelagert.

Die Rückstellvorrichtung (111) umfasst auch in diesem Ausführungsbeispiel einen Federenergiespeicher (115) in der Bauform einer Druckfeder (115). Diese ist beispielsweise so aufgebaut, wie im Zusammenhang mit dem ersten Ausführungsbeispiel beschrieben. Die Druckfeder (115) kann z.B. formschlüssig am Stützbund (84) und am Haltebund (34) fixiert sein. Beispielsweise kann der Applikator (10) dann ohne zusätzliche Auszugssicherung ausgeführt sein.

Anstatt als zylindrische Schrauben-Druckfeder kann der Federenergiespeicher (115) auch als Tellerfederpaket, als kegelige Schrauben-Druckfeder, etc. ausgebildet sein.

Das Schubgelenk (101) kann z.B. einen haptischen Indikator (116) aufweisen. Beispielsweise ist das Schubgelenk (101) so gestaltet, dass kurz vor dem Erreichen der Gebrauchsstellung (12) eine Rastnase überwunden werden muss. Der für den Bediener spürbare Widerstand wird beim Überwinden der Rastnase kurzfristig höher und sinkt dann schlagartig wieder ab. Die Rastnase kann unterschiedlich ausgebildete Flanken haben, sodass der Bediener beim versehentlichen Vermindern des Anpressdrucks ein anderes haptisches Signal erhält. Auch ein optischer oder ein akustischer Indikator (116) sind denkbar.

Bei der Anwendung des in der Figur 9 dargestellten Applikators (10) zieht der Bediener nach dem Entfernen der Abdeckfolie (136) den Applikator (10) über das Mikronadelpflaster (130). Hierbei drückt er den Applikator (10) auf das Mikronadelpflaster (130). Der Applikator (10) kann senkrecht oder schräg gehalten werden, wobei bei einer Schrägstellung das Griffstück (21) z.B. in Richtung der Pflasterlängsrichtung (135) geneigt ist. Der Applikator (10) wird dann gezogen. Die Druckfeder (115) wird komprimiert. Mittels des z.B. haptischen Signals wird der Bediener informiert, ob der Anpressdruck ausreichend ist oder erhöht werden muss.

Die Figuren 10 - 12 zeigen einen Applikator (10) mit einer integrierten Spendiervorrichtung (121). Das Griffstück (21) dieses Applikators (10) ist als Gehäuse (35) ausgebildet, in dem die Spendiervorrichtung (121) für ein Mikronadelpflaster (130) sowie eine Druckkörpergruppe (50) angeordnet sind.

Die Spendiervorrichtung (121) umfasst beispielsweise sechs zylindrisch ausgebildete Lagerrollen (122 - 125). Beispielsweise zwei dieser Lagerrollen (122, 123) sind oberhalb der Abdeckfolie (136) eines Mikronadelpflasters (130) und z.B. vier Lagerrollen (124, 125) unterhalb dieses Mikronadelpflasters (130) im Gehäuse (35) drehbar gelagert. Die Mikronadeln (132) des Mikronadelpflasters (130) zeigen nach unten. Die einzelnen Lagerrollen (122 - 125) können auch mittels eines Gestänges miteinander verbunden sein. Auch können die oberhalb und unterhalb des Mikronadelpflasters (130) einander gegenüberliegenden Lagerrollen (122, 124; 123, 125) z.B. mittels einer Zugfeder gegeneinander belastet sein. Die Drehachsen (126) aller Lagerrollen (122 - 125) sind parallel zueinander ausgerichtet. Die oberen Lagerrollen (122, 123) sind beispielsweise durchgehend ausgebildet. Ihre quer zur Pflasterlängsrichtung (135) orientierte Rollenlänge ist größer oder gleich der Breite des Mikronadelpflasters (130). Es ist aber auch denkbar, jede dieser oberen Lagerrollen (122; 123) durch zwei Einzelrollen zu ersetzen, deren Rollenlänge z.B. kürzer ist als die halbe Pflasterbreite.

Die unteren Lagerrollen (124, 125) sind Kurzrollen (124, 125). Ihre Länge in der Pflasterquerrichtung entspricht beispielsweise der Breite des seitlichen Überstands (138) des Überpflasters (133) über die Mikronadeln (132). Die Lagerrollen (122 - 125) können frei oder gebremst drehbar sein. Auch kann die Normalkraft der genannten Zugfeder den Roll- oder Wälzwiderstand der Lagerrollen (122 - 125) erhöhen.

Das Mikronadelpflaster (130) ist beispielsweise weitgehend so ausgebildet, wie im Zusammenhang mit den vorhergehenden Ausführungsbeispielen beschrieben. Im Gehäuse (35) schließt die Pflasterlängsrichtung (135) mit der Längsrichtung (25) des Griffstücks (21) beispielsweise einen Winkel zwischen 30 Grad und 60 Grad, z.B. 45 Grad, ein. Die Abdeckfolie (136) liegt z.B. vollflächig haftend auf dem Mikronadelpflaster (130) auf. Die Abdeckfolie (136) kann aber auch mit dem Mikronadelpflaster (130) verbunden sein, wie im Zusammenhang mit dem Ausführungsbeispiel der Figuren 5 - 8 beschrieben. An dem dem Druckkörper (51) zugewandten Ende sind in der Darstellung der Figur 10 das Überpflaster (133) und die Abdeckfolie (136) voneinander getrennt.

Die Druckkörpergruppe (50) ist in einem Schwenkgelenk (91) schwenkbar im Gehäuse (35) gelagert. Im Ausführungsbeispiel ist die Schwenkachse (95) des Schwenkgelenks (91) in der Drehachse (126) der ersten oberen Lagerrolle (122) angeordnet. Die Druckkörpergruppe (50) umfasst einen Druckkörper (51) und einen gabelförmig ausgebildeten Hebelarm (85). Dieser Hebelarm (85) ist im Schwenklager (91) gelagert. Im Ausführungsbeispiel trägt die Gabel (86) des Hebelarms (85) einen Achsstab (87), auf dem der als zylindrische Rolle ausgebildete Druckkörper (51) drehbar gelagert ist. Der Radius der Rolle (51) entspricht beispielsweise dem Radius des im Zusammenhang mit dem Ausführungsbeispiel der Figur 9 beschriebenen rollenförmigen Druckkörpers (51). Es ist aber auch denkbar, den Druckkörper (51) starr mit dem Hebelarm (85) zu verbinden. Beispielsweise kann die Hüllkontur des Druckkörpers (51) dann der Hüllkontur eines der im Zusammenhang mit den vorgenannten Ausführungsbeispielen beschriebenen nicht drehbaren Druckkörpern (51) entsprechen.

Der Applikator (10) hat eine im Gehäuse (35) angeordnete interne Rückstellvorrichtung (111). Diese umfasst einen Federenergiespeicher (115), der sich am Gehäuse (35) und an einem jochartig ausgebildeten Federteller (117) abstützt. Die Jocharme (118) des Federtellers (117) umgreifen den Druckkörper (51) an dessen Stirnseiten (69). Sie sind mit dem Achsstab (87) des Druckkörpers (51) verbunden. Beispielsweise sind sie am Achsstab (87) schwenkbar gelagert.

Der Federenergiespeicher (115) ist in diesem Ausführungsbeispiel als Druckfeder (115) ausgebildet. Es ist aber auch denkbar, den Federenergiespeicher (115) als Tellerfederpaket, als Zugfeder, etc. auszubilden.

Am Federteller (117) ist in den Darstellungen der Figuren 10 - 12 ein Zeigerarm (119) mit einem Indikator (116) angeordnet. Das Gehäuse (35) weist beispielsweise ein Sichtfenster (36) auf. Durch dieses hindurch ist der Indikator (116) in der Darstellung der Figur 10 unsichtbar. In den Darstellungen der Figuren 11 und 12, die den belasteten Applikator (10) zeigen, ist der Indikator (116) durch das Sichtfenster (36) hindurch sichtbar.

Am Gehäuse (35) ist weiterhin ein Haltehaken (37) befestigt. Dieser durchdringt und hintergreift den Federteller (117). Dieser Haltehaken (37) bildet eine Abhebesicherung der Rückstellvorrichtung (111).

Der Boden (38) des Gehäuses (35) hat eine Öffnung (39), durch die in der Darstellung der Figur 10 das Überpflaster (133) hindurchragt. Es ist auch denkbar, dass in der Versandlage des Applikators (10) die Öffnung (39) verschlossen ist. Das Mikronadelpflaster (130) wird dann beispielsweise mittels eines von der Außenseite des Gehäuses (35) betätigbaren Schiebers, einer Kurbel, etc. in die in der Figur 10 dargestellte Bereitschaftslage (13) gefördert.

Zwischen der Öffnung (39) und dem Druckkörper (51) ist im Gehäuseinnenraum (41) am Boden (38) ein Leitkeil (42) angeordnet. Dieser hat eine Trennkante (43) und eine an die Trennkante (43) angrenzende, in Richtung des Gehäuseinnenraums (41) orientierte Leitfläche (44). Die Leitfläche (44) kann beispielsweise so ausgebildet sein wie die obere Leitfläche (65) des in den Figuren 5 - 8 dargestellten Ausführungsbeispiels.

Beim Einsatz eines Druckkörpers (51) nach Figur 6 in einem Applikator (10) nach Figur 10 kann der Leitkeil (42) entfallen. Auch kann - bei einer formschlüssigen Verbindung zwischen Mikronadelpflaster (130) und Abdeckfolie (136) - die Druckkörpergruppe (50) aus dem Ausführungsbeispiel der Figuren 5 - 8 den Leitkeil (42) und den Druckkörper (51) ersetzen. Die Führungselemente (71) können hierbei auch gehäuseseitig angeordnet sein.

In der in der Figur 10 dargestellten Bereitschaftslage (13) liegt das Überpflaster (133) an der Andruckfläche (57) an. Die Abdeckfolie (136) ist bereichsweise entlang der Leitfläche (44) geführt. Der Druckkörper (51) steht relativ zum Gehäuse (35) in der Ruhestellung (11). Die Rückstellvorrichtung (111) ist entlastet.

Beim Andrücken und Ziehen des Applikators (10) entlang der Haut (1) wird das Mikronadelpflaster (130) aus der Spendiervorrichtung (121) herausgezogen, vgl. Figur 11. Der Druckkörper (51) wird in Richtung der Gebrauchslage (12) eingedrückt, wobei die Rückstellvorrichtung (111) belastet wird. Der Indikator (116) wird im Sichtfenster (36) sichtbar. Mittels des Druckkörpers (51) wird das Mikronadelpflaster (130) auf die Haut (1) des Patienten gepresst, wobei die Mikronadeln (132) in die Haut (1) eingedrückt werden. Der Druckkörper (51) wälzt hierbei auf dem Mikronadelpflaster (130) ab. Beim weiteren Ziehen des Applikators (10) in der Zugrichtung (15) werden sämtliche Mikronadeln (132) eingepresst. Nach vollständigem Abwälzen auf dem Mikronadelpflaster (130) ist das Mikronadelpflaster (130) auf der Haut (1) fixiert, vgl. Figur 12.

Die genannten Ausführungsbeispiele können auch miteinander kombiniert werden.

## Patentansprüche

1. Applikator (10) mit einem Griffstück (21) und mit einem mit dem Griffstück (21) verbundenen Druckkörper (51) für ein Mikronadelpflaster (130),
**dadurch gekennzeichnet,**
- **dass** der Druckkörper (51) relativ zum Griffstück (21) in einem Schubgelenk (101) oder in einem Schwenkgelenk (91) zumindest zwischen einer Ruhestellung (11) und einer Gebrauchsstellung (12) mittels äußerer Betriebskräfte und einer internen Rückstellvorrichtung (111) verstellbar gelagert ist,
- **dass** der Druckkörper (51) eine vom Schwenkgelenk (91) oder Schubgelenk (101) beabstandete Andruckfläche (57) aufweist,
- **dass** in der Ruhestellung (11) der Druckkörper (51) von äußeren Betriebskräften unbelastet ist und die Rückstellvorrichtung (111) die geringste innere Energie aufweist und
- **dass** der Druckkörper (51) mittels an der Andruckfläche (57) angreifenden äußeren Betriebskräften relativ zum Griffstück (21) in Richtung der Gebrauchsstellung (12) unter Erhöhung der inneren Energie der Rückstellvorrichtung (111) verstellbar ist.

2. Applikator (10) nach Anspruch 1,
**dadurch gekennzeichnet,**
**dass** ein Sicherungselement (61, 96) den Verstellbereich in der Ruhestellung (11) begrenzt.

3. Applikator (10) nach Anspruch 1,
**dadurch gekennzeichnet,**
**dass** der Druckkörper (51) oder eine den Druckkörper (51) beinhaltende Druckkörpergruppe (50) am oder im Griffstück (21) gelagert ist.

4. Applikator (10) nach Anspruch 1,
**dadurch gekennzeichnet,**
**dass** die Rückstellvorrichtung (111) einen belastungsabhängigen optischen, haptischen oder akustischen Indikator (116) aufweist,

5. Applikator (10) nach Anspruch 1,
**dadurch gekennzeichnet,**
**dass** die Andruckfläche (57) eine stetig gewölbte Fläche ist, deren mindestens eine Wölbungsradius-Mittellinie mit dem kleinsten Radius zumindest in der Gebrauchsstellung (12) normal zu einer Längsrichtung (25) und windschief zu einer Längsachse (33) des Griffstücks (21) ist.

6. Einheit (150) aus einem Applikator (10) nach Anspruch 1 und aus einem Mikronadelpflaster (130) mit einer Vielzahl von Mikronadeln (132),
**dadurch gekennzeichnet,**
**dass** der kleinste Radius der Andruckfläche (57) mindestens dem anderthalbfachen Produkt aus der Kreiszahl π und der Länge des Nadelfußes (137) einer Mikronadel (132) in der Pflasterlängsrichtung (135) beträgt.

7. Einheit (150) nach Anspruch 6,
**dadurch gekennzeichnet,**
**dass** das Mikronadelpflaster (130) zumindest bereichsweise eine Abdeckfolie (136) trägt und dass der Applikator (10) eine keilförmige Trennkante (43; 66) mit einer daran angrenzenden Leitfläche (44; 65) zum Ableiten der Abdeckfolie (136) aufweist.

8. Einheit (150) nach Anspruch 7,
**dadurch gekennzeichnet,**
**dass** die Trennkante (66) am Druckkörper (51) ausgebildet ist.

9. Einheit (150) nach Anspruch 8,
**dadurch gekennzeichnet,**
**dass** am Druckkörper (51) Führungselemente (71) angeordnet sind, die zumindest ein Überpflaster (133) des Mikronadelpflasters (130) und die Abdeckfolie (136) übergreifen.

## Claims

1. Applicator (10) having a handle piece (21) and having a pressing body (51) for a microneedle patch (130), which pressing body (51) is connected to the handle piece (21),
**characterized in that**
- the pressing body (51) is mounted for movement relative to the handle piece (21) in a sliding joint (101) or in a pivot joint (91) at least between an idle position (11) and a usage position (12) by means of external operating forces and an internal restoring device (111),
- the pressing body (51) has a pressing surface (57) which is spaced apart from the pivot joint (91) or sliding joint (101),
- in the idle position (11) the pressing body (51) is not loaded by external operating forces and the restoring device (111) has the lowest internal energy, and
- the pressing body (51) is movable relative to the handle piece (21) towards the usage position (12) by means of external operating forces acting on the pressing surface (57), the internal energy of the restoring device (111) thus being increased.

2. Applicator (10) according to claim 1,
**characterized in that**
a securing element (61, 96) limits the range of movement in the idle position (11).

3. Applicator (10) according to claim 1,
**characterized in that**
the pressing body (51) or a pressing body group (50) containing the pressing body (51) is mounted on or in the handle piece (21).

4. Applicator (10) according to claim 1,
**characterized in that**
the restoring device (111) has a load-dependent visual, haptic or acoustic indicator (116).

5. Applicator (10) according to claim 1,
**characterized in that**
the pressing surface (57) is a continuously curved surface, of which the at least one radius of curvature center line with the smallest radius is normal to a longitudinal direction (25) and skew to a longitudinal axis (33) of the handle piece (21) at least in the usage position (12).

6. Unit (150) comprising an applicator (10) according to claim 1 and comprising a microneedle patch (130) having a plurality of microneedles (132),
**characterized in that**
the smallest radius of the pressing surface (57) is at least one and a half times the product of the circle constant n and the length of the heel (137) of a microneedle (132) in the patch's longitudinal direction (135).

7. Unit (150) according to claim 6,
**characterized in that**
the microneedle patch (130) carries a cover film (136) at least in part and **in that** the applicator (10) has a wedge-shaped separating edge (43; 66) with an adjacent guiding surface (44; 65) for guiding away the cover film (136).

8. Unit (150) according to claim 7,
**characterized in that**
the separating edge (66) is formed on the pressing body (51).

9. Unit (150) according to claim 8,
**characterized in that**
guide elements (71) are arranged on the pressing body (51), which guide elements (71) engage at least over an overpatch (133) of the microneedle patch (130) and over the cover film (136).

## Revendications

1. Applicateur (10) doté d'une pièce de préhension (21) et d'un corps de pression (51) relié à la pièce de préhension (21) pour un timbre à micro-aiguilles (130),
**caractérisé**
- **en ce que** le corps de pression (51) est disposé de manière réglable au moins entre une position de repos (11) et une position d'utilisation (12) relativement à la pièce de préhension (21) dans un joint à glissière (101) ou dans un joint pivotant (91), au moyen de forces de fonctionnement extérieures et d'un dispositif de rappel interne (111),
- **en ce que** le corps de pression (51) comporte une surface d'appui (57) à distance du joint pivotant (91) ou du joint à glissière (101),
- **en ce que** dans la position de repos (11), le corps de pression (51) n'est pas contraint par des forces de fonctionnement extérieures et le dispositif de rappel (111) comporte l'énergie interne la plus petite et
- **en ce que** le corps de pression (51) est réglable par rapport à la pièce de préhension (21) en direction de la position d'utilisation (12) au moyen de forces de fonctionnement extérieures agissant sur la surface d'appui (57), en augmentant l'énergie interne du dispositif de rappel (111).

2. Applicateur (10) selon la revendication 1, **caractérisé en ce qu'**un élément d'immobilisation (61, 96) délimite la plage de réglage dans la position de repos (11) .

3. Applicateur (10) selon la revendication 1, **caractérisé en ce que** le corps de pression (51) ou un groupe de corps de pression (50) comprenant le corps de pression (51) est monté sur ou dans la pièce de préhension (21).

4. Applicateur (10) selon la revendication 1, **caractérisé en ce que** le dispositif de rappel (111) comporte un indicateur (116) optique, haptique ou acoustique dépendant de la contrainte.

5. Applicateur (10) selon la revendication 1, **caractérisé en ce que** la surface d'appui (57) est une surface recourbée en continu, dont au moins une médiane de rayon de courbure avec le plus petit rayon est, au moins dans la position d'utilisation (12), normale à une direction longitudinale (25) et en oblique par rapport à un axe longitudinal (33) de la pièce de préhension (21).

6. Unité (150) constituée d'un applicateur (10) selon la revendication 1 et d'un timbre à micro-aiguilles (130) doté d'une pluralité de micro-aiguilles (132), **caractérisée en ce que** le plus petit rayon de la surface d'appui (57) est au moins égal à une fois et demi le produit de π et de la longueur du talon (137) d'une micro-aiguille (132) dans la direction longitudinale du timbre (135).

7. Unité (150) selon la revendication 6, **caractérisée en ce que** le timbre à micro-aiguilles (130) porte au moins par endroits un film de masquage (136) et **en ce que** l'applicateur (10) comporte un bord de séparation en forme de coin (43 ; 66) doté d'une surface déflectrice adjacente (44 ; 65) destinée à enlever le film de masquage (136).

8. Unité (150) selon la revendication 7, **caractérisée en ce que** le bord de séparation (66) est réalisé sur le corps de pression (51).

9. Unité (150) selon la revendication 8, **caractérisée en ce que** des éléments de guidage (71) sont disposés sur le corps de pression (51), lesquels viennent en prise sur au moins un surtimbre (133) du timbre à micro-aiguilles (130) et sur le film de masquage (136).
